# EUROPEAN PATENT SPECIFICATION

(11) **EP 2 552 321 B1**
(45) Date of publication and mention of the grant of the patent: **12.03.2014**
(21) Application number: 11728697.1
(22) Date of filing: 01.04.2011
(51) Int. Cl.: A61B 10/00

(54) **AUXILIARY DEVICE FOR RECEIVING FECAL MATTER**
HILFSVORRICHTUNG ZUR AUFNAHME VON FÄKALIEN
DISPOSITIF AUXILIAIRE DESTINÉ À RECEVOIR DES MATIÈRES FÉCALES

(30) Priority: 02.04.2010 NL 2004507
(43) Date of publication of application: 06.02.2013
(73) Proprietor: Bosch, Antonius Henricus Petrus, 5671 CS Nuenen (NL)
(72) Inventor: Bosch, Antonius Henricus Petrus, 5671 CS Nuenen (NL)
(74) Representative: Verhees, Godefridus Josephus Maria
(86) International application number: PCT/NL2011/050220
(87) International publication number: WO 2011/122949

(56) References cited:
- WO-A1-03/088842
- WO-A1-2005/014154
- WO-A2-02/15791
- US-A- 3 754 287
- US-A1- 2003 060 787
- US-A1- 2007 245 486

## Description

### Field of the invention.

The invention relates to an auxiliary device for receving fecal matter, comprising a container open at the top, as well as a removable cover present on the container and closing off the top side of the container, and **a** frame provided with arms with which the frame can rest on the rim of a toilet bowl, as well as an opening in which the container can be accommodated.

### State of the art.

An auxiliary device of this type is known from US 3 754 287 A. During use the container floats in water present in a toilet bowl and the container is kept at the right place by the limiting rim of the opening. The shape of the frame may be obtained by folding a pre-folded plate along folding lines introduced beforehand. As a result, the frame can in a simple manner be packed as a set together with the container.

Since the plate the frame is made of is longer than the width of a toilet bowl and the container is to be sufficiently high to receive a sufficient amount of fecal matter, the set will be relatively voluminous and have large dimensions. In consequence, storage and transportation of a large number of these sets requires much space. Since this auxiliary device is especially intended for preventive population screening, vast quantities will be needed with consequential high storage, transportation and forwarding costs.

### Summary of the invention.

It is an object of the invention to provide an auxiliary device for receiving fecal matter of the type defined in the opening paragraph which can be transported in a simpler manner and takes up less space during storage and transportation than the known auxiliary device. For this purpose the auxiliary device according to the invention is characterised in that the frame comprises a container-retaining carrier present around the opening, where the arms are hingeably connected to the carrier and in a folded state are present underneath the carrier and in an unfolded state rest against a stop that prevents further unfolding, and where the container is extractable and is present in a compressed state and can be drawn out before use. As a result, the auxiliary device is rather compact before use and takes up little space during storage and transportation and can be dispatched in a simple manner.

The side wall of the container is preferably accordion-shaped and in extracted state the container is stable, which is to say that the side wall remains erect and does not sag by itself.

The carrier is preferably formed by a ring on which the container rests with a protruding rim and in which the container can be installed and clamped. In consequence, the container retains its desired position during use and can afterwards be removed from the frame in a simple manner.

### Brief description of the drawings.

The following description relating to the appended drawings, the whole given by way of non-limiting example of the auxiliary device according to the invention, will provide better understanding of how the invention can be realised, in which:
Fig. 1 shows the auxiliary device in a folded state seen from above;
Fig. 2 shows the auxiliary device shown in Fig. 1 seen from below;
Fig. 3 shows the auxiliary device with extracted container seen from above;
Fig. 4 shows the auxiliary device shown in Fig. 3 seen from below;
Fig. 5 shows the auxiliary device with unfolded arms;
Fig. 6 shows the auxiliary device with unfolded arms and removed cover;
Fig. 7 shows the auxiliary device mounted on a toilet bowl with removed cover; and
Fig. 8 shows the auxiliary device mounted on a toilet bowl closed off by the cover.

### Detailed description of the drawings

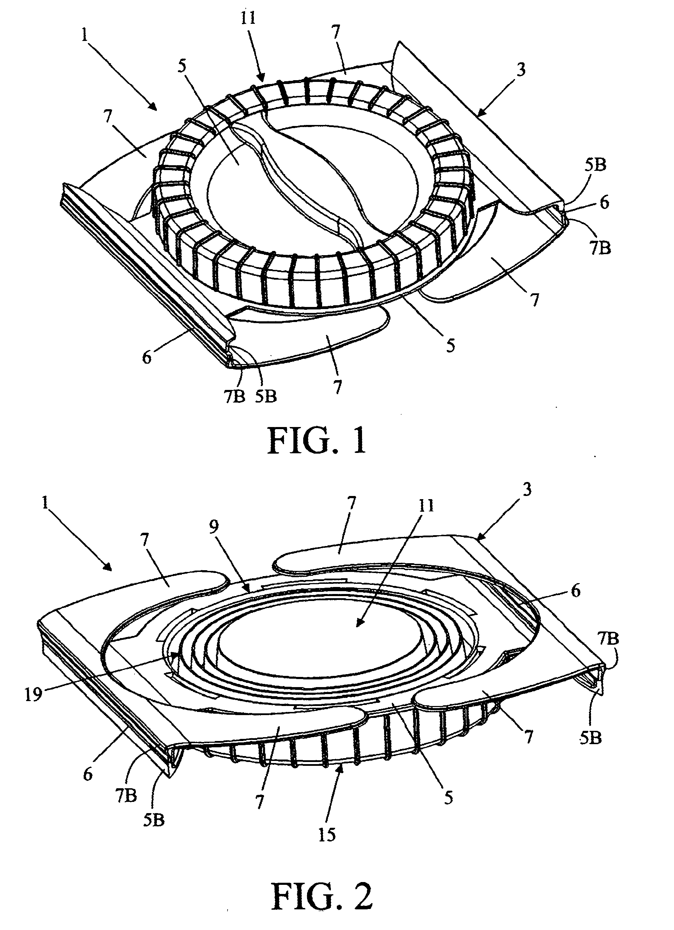

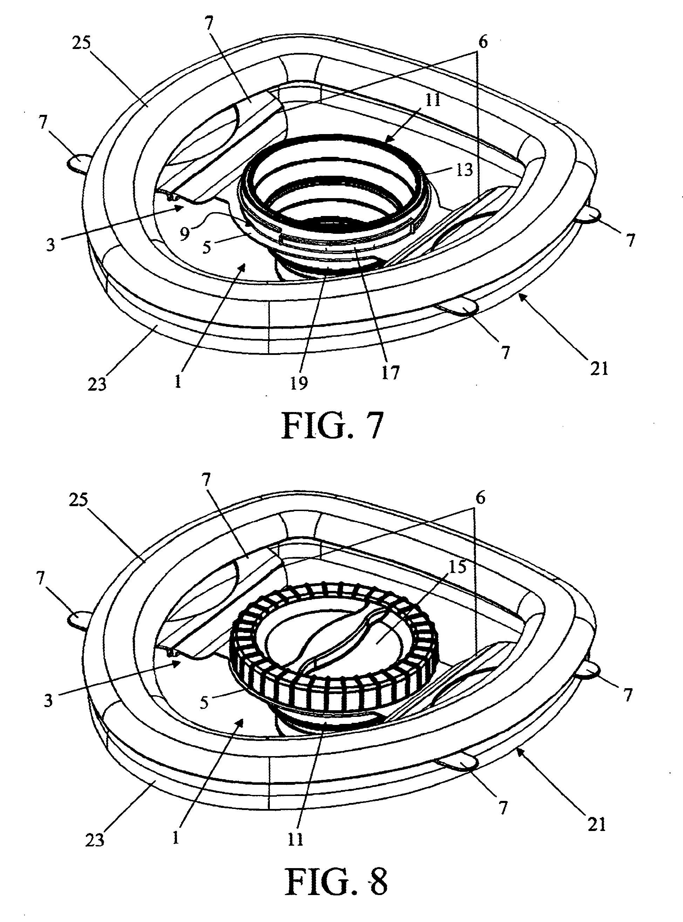

Figs. 1 and 2 show an embodiment of the folded state auxiliary device according to the invention for receiving fecal matter. The auxiliary device 1 comprises a frame 3 which is made of plastic as a whole. This frame comprises a carrier 5 and arms 7 connected thereto by means of flexible hinges 6. The arms are interlinked in pairs and in folded state are located underneath the carrier 5. The carrier 5 is formed by a ring having an opening 9 in the middle (cf. Fig. 7).

The interlinked parts 5B and 7B of the carrier 5 and arms 7 are at right angles to the rest of the carrier and arms. At their free edges these parts are linked to each other by means of flexible hinges 6. The arms 7 can be unfolded until the parts 5B and 7B hit each other. These parts form stops that limit the angle over which the arms can be unfolded In unfolded state the arms and the carrier lie in one plane.

The auxiliary device I further includes a container 11 open at the top 13 (cf. Fig. 7), which container is closed off by a removable cover 15 which is already present in the opening 9. The container 11 rests with a protruding rim 17 present at the top (cf. Fig. 7) on the carrier 5 and is hence confined to the ring in a clamping manner. The clamping effect is such that the container 11 can be removed from the carrier 5.

The container 11 is present in a collapsed state and is to be extracted before use. The side wall 19 (cf. Fig. 7) of the container is accordeon-shaped and is stable in an extracted state.

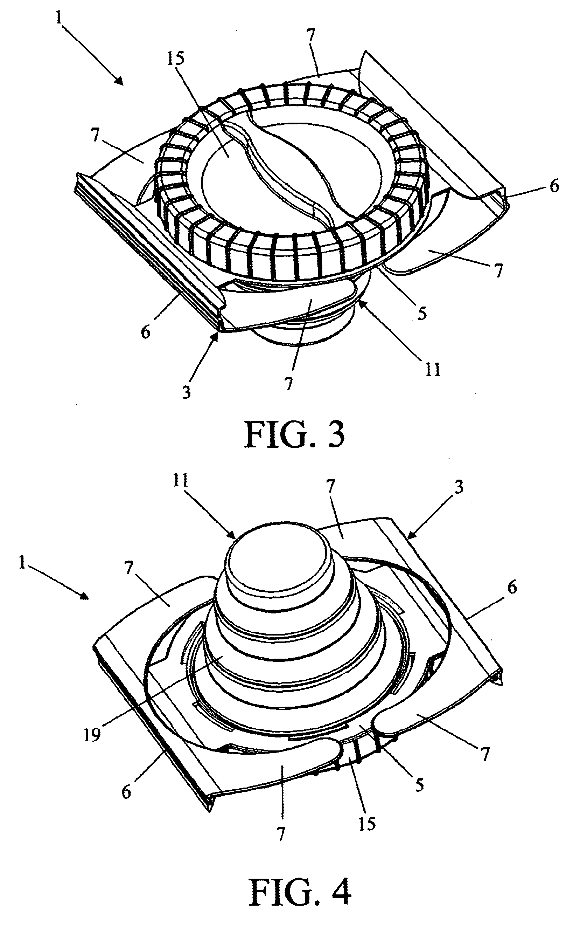

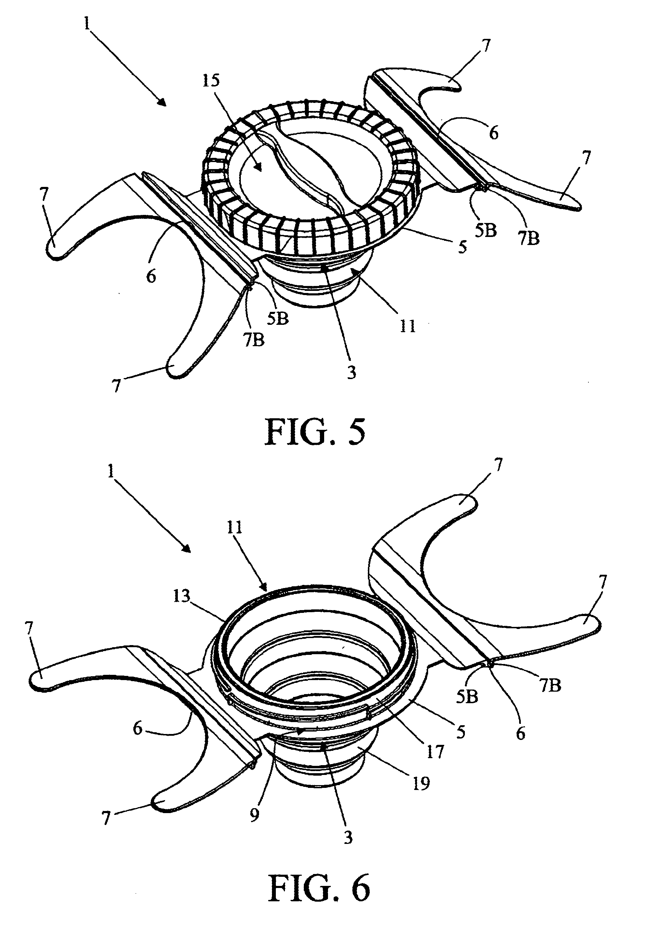

For illustrative purposes Figs. 3 and 4 show the auxiliary device 1 with extracted container 11 while Figs. 5 and 6 show the auxiliary device 1 with unfolded arms 7.

During use the auxiliary device 1 rests with its arms 7 on the rim 23 of a toilet bowl 21, cf. Figs. 7 and 8. The arms 7 are positioned between the rim 23 of the toilet bowl and the toilet seat 25. After use the cover 15 is put on the container 11 after which the container can be removed from the frame 3.

Albeit the invention has been described in the foregoing with reference to the drawings, it should be pointed out that the invention is not by any manner or means restricted to the embodiments shown in the drawings. The invention also extends over any embodiments deviating from the embodiments shown in the drawing Figures within the scope defined by the claims.

## Claims

1. An auxiliary device (1) for receving fecal matter, comprising a container (11) open at the top (13), as well as a removable cover (15) present on the container and closing off the top side of the container, and a frame (3) provided with arms (7) with which the frame can rest on the rim of a toilet bowl, as well as an opening (9) in which the container can be accommodated, **characterised in that** the frame (3) comprises a carrier (5) present around the opening (9) and retaining the container (11), where the arms are hingeably connected to the carrier and in a folded state are present underneath the carrier and in an unfolded state rest against a stop (5B) that prevents further unfolding, and where the container is extractable and is present in a compressed state and can be extracted before use.

2. An auxiliary device (1) as claimed in claim 1, **characterised in that** the side wall (19) of the container is accordeon-shaped and is stable in an extracted state.

3. An auxiliary device (1) as claimed in claim 1 or 2, **characterised in that** the carrier (5) is formed by a ring on which the container (11) rests with a protruding rim (17) and in which the container can be installed and clamped.

## Patentansprüche

1. Hilfsmittel (1) zur Aufnahme von Stuhlgang, bestehend aus einem an der Oberseite (13) offenen Behälter (11), sowie einem entfernbaren Deckel (15) auf dem Behälter, der die Oberseite des Behälters verschließt, und einem Rahmen (3) mit Armen (7), mit denen der Rahmen auf dem Toilettenbecken ruhen kann, sowie einer Öffnung (9), in die der Behälter eingesetzt werden kann, **dadurch gekennzeichnet, dass** der Rahmen (3) ein rund um die Öffnung (9) vorhandenes Tragelement (5) enthält, das den Behälter (11) arretiert, wobei die Arme drehbar mit dem Tragelement verbunden sind und sich im eingeklappten Zustand unter dem Tragelement befinden und im ausgeklappten Zustand an einem Anschlag (5B) anliegen, der ein weiteres Ausklappen verhindert, wobei der Behälter ausziehbar und in einem ineinander gedrückten Zustand vorhanden ist und zur Benutzung auseinander gezogen werden kann.

2. Hilfsmittel (1) nach Anspruch 1, **dadurch gekennzeichnet, dass** die Seitenwand (19) des Behälters ziehharmonikaartig und im ausgezogenen Zustand stabil ist.

3. Hilfsmitte (1) nach Anspruch 1 oder 2, **dadurch gekennzeichnet, dass** das Tragelement (5) durch einen Ring gebildet wird, auf dem der Behälter (11) mit einem hervorstehenden Rand (17) ruht und auf dem der Behälter geklemmt werden kann.

## Revendications

1. Outil d'aide (1) pour la réception de selles, comprenant un conteneur (11) ouvert sur le dessus (13), ainsi qu'un couvercle (15) amovible présent sur le conteneur et fermant le dessus du conteneur, et un châssis (3) pourvu de bras (7) grâce auxquels le châssis peut reposer sur le bord d'une cuvette de toilette, ainsi qu'une ouverture (9) dans laquelle peut être placé le conteneur, **caractérisé en ce que** le châssis (3) comprend un support (5) qui se trouve autour de l'ouverture (9) et qui maintient le conteneur (11), où les bras sont articulés et sont reliés au support, et en position repliée se trouvent sous le support, et en position dépliée sont adjacents à une butée (5B) qui empêche un plus grand déploiement, et où le conteneur est extractible et se trouve dans une position comprimée et peut être étiré lors de l'utilisation.

2. Outil d'aide (1) selon la revendication 1, **caractérisé en ce que** la paroi latérale (19) du conteneur est en forme de soufflet et est stable en position rentrée.

3. Outil d'aide (1) selon la revendication 1 ou 2, **caractérisé en ce que** le support (5) est formé d'un anneau sur lequel repose le conteneur (11) en son bord (17) qui dépasse, et dans lequel le conteneur peut être immobilisé.
